(19) **Europäisches Patentamt**

**European Patent Office**

**Office européen des brevets**

(11) Publication number: **0 653 215 A1**

(12) **EUROPEAN PATENT APPLICATION**
**published in accordance with Art. 158(3) EPC**

(21) Application number: **94919891.5**

(22) Date of filing: **12.07.94**

(86) International application number:
**PCT/JP94/01136**

(87) International publication number:
**WO 95/02425 (26.01.95 95/05)**

(51) Int. Cl.6: **A61M 1/34**

(30) Priority: **12.07.93 JP 171759/93**

(43) Date of publication of application:
**17.05.95 Bulletin 95/20**

(84) Designated Contracting States:
**AT BE CH DE DK ES FR GB GR IE IT LI LU MC NL PT SE**

(71) Applicant: **OTSUKA PHARMACEUTICAL FACTORY, INC.**
**115, Kugahara,**
**Tateiwa,**
**Muyacho**
**Naruto-shi**
**Tokushima 772 (JP)**

(72) Inventor: **KAMOGAWA, Hiroshi**
**1-8, Aza Hikata**
**Shinkirai**

**Kitajima-cho**
**Itano-gun**
**Tokushima 771-02 (JP)**
Inventor: **SUGIOKA, Yoshihiro**
**21, Minamimaekawacho 2-chome**
**Tokushima-shi**
**Tokushima 770 (JP)**
Inventor: **FURUYA, Akihiko**
**336-2, Nishikashiwabarashinden**
**Fuji-shi**
**Shizuoka 417 (JP)**

(74) Representative: **Gudel, Diether, Dr.**
**Patentanwälte**
**Dr. Weinhold, Dannenberg,**
**Dr. Gudel, Schubert**
**Grosse Eschenheimer Strasse 39**
**D-60313 Frankfurt (DE)**

(54) **COOLER FOR BLOOD PLASMA COOLING BAGS.**

(57) A cooler for blood plasma cooling bags according to the present invention has a plurality of thermoelectric cooling units arranged in a scattered state between a cooling plate and a radiating plate, a blood plasma bag being cooled with the cooling plate, the radiation of heat being carried out by the radiating fins provided on the radiating plate and a radiating fan for sending air to these fins, the cooler being characterized by: (i) a clearance of at least 5 mm being provided between the cooling plate and radiating plate; (ii) the thermoelectric cooling units being joined at their heat absorption sides to the cooling plate via a heat conductive block, and at their radiation sides to the radiating plate directly, or the thermoelectric cooling units being joined at their heat absorption sides to the cooling plate directly, and at their radiation sides to the radiating plate via a heat conductive block; (iii) the clearance between the cooling plate and radiating plate being filled with a heat insulating material, the cooler enabling the time required to attain a set temperature to be reduced, and the set temperature maintaining stability to be improved.

FIG. 2

TECHNICAL FIELD

The present invention relates to a cooler useful for cooling plasma cooling bags, especially a plasma cooling bag constituting a plasma cooling portion which is provided at an intermediate portion of a plasma line for practicing cryofiltration.

BACKGROUND ART

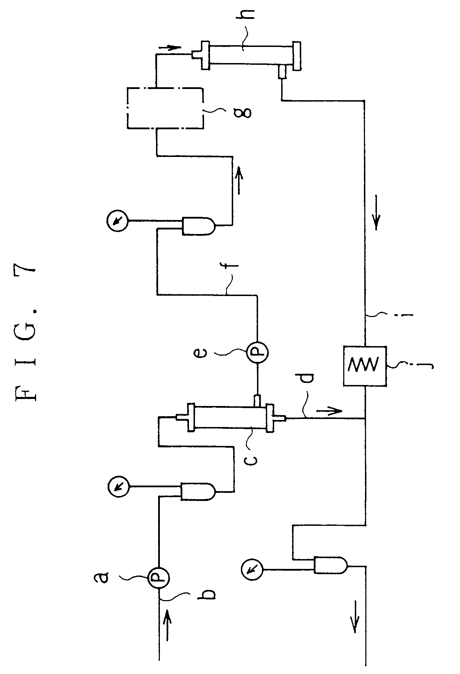

Cryofiltration is a therapy wherein the plasma separated from the blood of the patient is cooled to filter off a gellike substance (pathogenic substance) produced on cooling. This therapy is effective, for example, for patients with rheumatism and has therefore attracted attention. Cryofiltration will be described with reference to FIG. 7. By the operation of a blood pump a, blood is sent from a supply source thereof through a blood supply line b to a primary filter C, which separates the blood into blood cells and plasma. The separated blood cells are sent to a blood cell return line d so as to be mixed again with the purified plasma to be described below. On the other hand, the plasma is sent by the operation of a plasma pump e from a plasma line f into a secondary filter h for filtration by way of a cooling portion g on the line f. The plasma is cooled by the cooling portion g to about 4 C°, and a cryogel of high-molecular-weight substance produced by the cooling is filtered off by the secondary filter and thereby removed from the plasma. The filtrate from the secondary filter, i.e., purified plasma, is remixed with the blood cells returned from the foregoing primary filter while being returned through a purified plasma return line i. The mixture is thereafter returned to the supply source while being heated to the initial liquid temperature by a heating bag j.

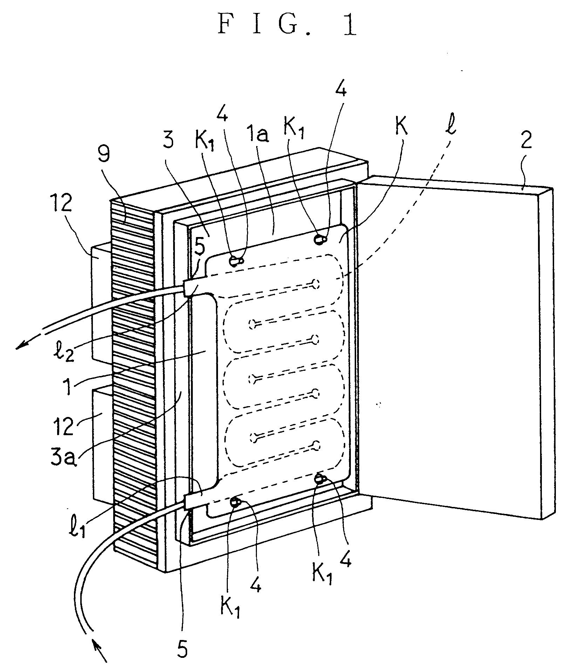

In recent years, a method has been proposed of cooling plasma in the cooling portion g by using a constant-temperature chamber of the refrigerant (e.g., antifreeze solution) circulation type for cooling the plasma while the plasma flows through a zigzag channel l of a disposable plasma cooling bag k (see FIG. 1). This method has the following problems.

(1) Difficulty in temperature control.

It is not easy to maintain a constant temperature. Further it takes time for the plasma to reach the predetermined temperature. Since the refrigerant is made usually about 5° lower than the desired temperature, the plasma is excessively cooled when the circulation of plasma is temporarily interrupted.

(2) The system becomes large-sized.

The constant-temperature chamber is difficult to make compact, consequently rendering the overall plasma cryofiltration system large and difficult to move.

(3) Likelihood of soiling and malfunction.

The refrigerant splashes to wet the operator or the neighborhood of the chamber or to expose the system to the refrigerant, causing a malfunction.

It has also been proposed to use a cooler incorporating electronic cooling units as cooling means substituting for the constant-temperature chamber (U.S. Patent No. 4,872,978). The problems (1) to (3) of the chamber can be overcome by this cooling means. However, the proposed device is adapted to cool a container disposed upstream from the secondary filter and temporarily storing plasma, and therefore has the drawback that the container can not be compacted in reducing the size of the device, necessitating an increased priming volume. Additionally, no consideration is given to the arrangement of electronic cooling units, so that the device is not suitable for cooling a flow of plasma although serviceable for cooling the stored plasma. More specifically, if the proposed device is used as it is for the plasma cooling bag, the device, which has a low cooling capacity, requires time for cooling plasma to a predetermined temperature and encounters difficulty in maintaining the predetermined temperature. For example, when plasma having a temperature approximate to the body temperature was passed through the bag at a flow rate of about 30 ml/min, it was substantially impossible for the device to cool the plasma to 4 °C. Accordingly, there arises a need to use measures such as larger radiator fins or radiator fans, an increased number of electronic cooling units, a water-cooled radiator portion and a plasma cooling bag having an elongated channel. These measures nevertheless give rise to other problems such as (a) not permitting compaction of the device, (b) an increased cost, (c) difficulty in moving the device, and (d) an increased priming volume. Thus, much still remains to be improved before the actual use of the device.

On the other hand, U.S. Patent No. 3,399,536 proposes a device wherein electronic cooling units are employed for altering the temperature of blood for use in surgery. The proposal, however, entirely differs from the present invention in object and includes no contrivance for ensuring a high cooling capacity for cryofiltration. Accordingly, the device is not usable actually as it is for the plasma cooling bag because the same problems as above will arise.

DISCLOSURE OF THE INVENTION

The main object of the present invention is to obviate all the foregoing problems (a) to (d) of the coolers comprising electronic cooling units and to provide a compact and efficient cooler of this type.

Other objects of the invention will be made apparent from the following description.

The invention provides a cooler for plasma cooling bags which comprises a multiplicity of electronic cooling units dispersedly arranged between a cooling plate and a radiating plate for cooling the plasma cooling bag by the cooling plate and removing heat from the radiating plate by radiating fins provided on the radiating plate and a radiating fan for applying a current of air to the fins, the cooler being characterized in that:

(i) a space of at least 5 mm is provided between the cooling plate and the radiating plate,

(ii) the electronic cooling units have a heat absorbing side fastened to the cooling plate with a thermally conductive block interposed therebetween and a radiating side joined directly to the radiating plate, or the electronic cooling units have their heat absorbing side joined directly to the cooling plate and their radiating side fastened to the radiating plate with a thermally conductive block interposed therebetween , and

(iii) a heat insulating material is filled in a clearance between the cooling plate and the radiating plate.

BRIEF DESCRIPTION OF THE DRAWINGS

FIG. 1 is a perspective view schematically showing an embodiment of the invention;

FIG. 2 is a view in central vertical section of the same;

FIG. 3 is a diagram for illustrating an arrangement of electronic cooling units according to the invention;

FIG. 4 is a schematic diagram of a comparative device ;

FIG. 5 is a diagram for illustrating an arrangement of electronic cooling units in the comparative device;

FIG. 6 is a graph showing the result of a cooling efficiency comparative test; and

FIG. 7 is a diagram for illustrating cryofiltration.

BEST MODE OF CARRYING OUT THE INVENTION

An embodiment of the invention will be described below with reference to the accompanying drawings.

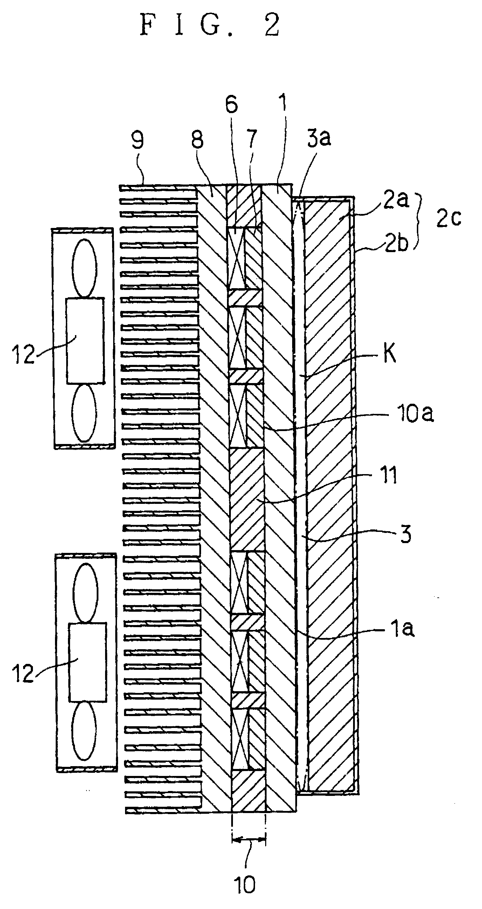

The construction of cooler of the invention will be described with reference to the sketch of FIG. 1 schematically showing the same and based on the vertical sectional view of FIG. 2.

The cooler of the invention has a cooling plate 1 made of a metal of high thermal conductivity, such as aluminum, and usually about 5 mm in thickness. The material and thickness of the plate are not limitative but may be selectively determined suitably.

The cooling plate 1 is provided on its front side with a receptacle portion 3 into which a plasma cooling bag k can be removably placed when a heat insulating lid 2 is opened. The receptacle portion 3 has inside thereof a cooling face la which is provided, for example, with two pairs of right and left bag fastening hooks 4 (see FIG. 1) at upper and lower two portions, respectively, for holding the accommodated bag k generally in face-to-face contact with the cooling face la. In this case, the bag k is formed at its upper and lower ends with hook engaging holes $k_1$ as positioned in corresponding relation with the respective hooks 4. Preferably, the plasma cooling bag k is flat and flexible and has an elongated zigzag channel l.

A frame 3a serving as the side wall of the receptacle portion 3 has cutouts 5, 5 for receiving connectors $l_1$, $l_2$ for holding a plasma line f in communication with the inlet and outlet of the channel l of the bag k as accommodated in place. This assures that the connectors $l_1$, $l_2$ provide a plasma passage (not shown) therethrough free of trouble while the heat insulating lid 2 is closed.

The heat insulating lid 2 can be made up of a body 2a of heat insulating material, and a cover 2b provided over the side periphery and outer surface of the body 2a. Incidentally, the lid 2 also serves to press the bag k against the cooling face la to enhance the intimate contact therebetween.

The external exposed portion of front surface of the cooling plate 1 and the external exposed portion of the frame 3a can be provided with a heat insulating cover.

Electronic cooling units 6 for absorbing heat from the cooling plate 1 are disposed on the rear side of the plate 1.

The electronic cooling units 6 operate on the principle of the Peltier effect for electronic cooling and are manufactured and made commercially available by Thermovonics Co., Ltd. of Japan.

The cooling unit 6, which is commercially available, is in the form of a square measuring about 5 to about 40 mm in the length of one side or a rectangle similar to the square, and has a thickness of about 2.0 to about 4.5 mm.

A multiplicity of, for example, about 5 to about 15, such electronic cooling units 6 ate dispersedly arranged on the rear side of the cooling plate 1. The number of units 6 to be installed and the spacing therebetween are suitably determined, for example, in accordance with the cooling efficiency of the unit 6 and the size of the cooling plate 1. Use of a larger number of units naturally results in a higher cooling capacity but also increases the cost of the device itself and power consumption, so that the number of units to be installed is preferably smaller. To exploit the performance of the electronic cooling units to the greatest possible extent, it is therefore desirable to arrange at least some of the units in the region to be exposed to a current of air applied to the radiating plate by a radiating fan. Furthermore, it is especially suitable that the total of the areas occupied by the electronic cooling units in the region be at least 10%, preferably 10 to 20%, of the area of the cooling plate. Generally, the cooling plate 1 approximately measures 30 cm (long) x 20 cm (wide).

Each of the electronic cooling units 6 has a heat absorbing face to which a thermally conductive block 7 is fitted in register therewith. The block 7 is in face-to-face contact with the rear surface of the cooling plate 1.

The block 7 is made of a metal of high thermal conductivity, such as copper, pure silver or aluminum. The thickness of the block 7 is determined from such a range that the sum of the thickness and that of the unit 6 is at least 5 mm. When the unit 6 is, for example, 3 mm in thickness, it is suitable that the thickness be about 2 to about 4 mm. The block 7 is preferably at least 0.8 cal/cm•sec•deg, more preferably 0.9 cal/cm•sec•deg, in thermal conductivity.

The electronic cooling units 6 each have a radiating face in direct face-to-face contact with a radiating plate 8. The radiating plate 8 is nearly identical with the cooling plate 1 in shape and size, and opposed to the plate 1 face-to-face in register therewith.

The radiating plate 8 is provided with radiating fins 9 on its rear side. The total radiating area of the radiating fins 9 is at least three times the area of one surface of the cooling plate 1. Like the cooling plate 1 and blocks 7, the radiating fins 9 are made of a material of high thermal conductivity.

The electronic cooling units 6 and the respective blocks 7 superposed thereon serve as spacers between the cooling plate 1 and the radiating plate 8, providing between the two plates 1 and 8 a space 10 of at least 5 mm corresponding to the total thickness of the unit and the block.

The space 10 includes around the units 6 and the blocks 7 a clearance l0a which is filled with a heat insulating material 11. A polyurethane foam having good heat insulating properties is suitable as the heat insulating material 11. The heat insulating material 11 is preferably up to 0.03 kcal/m•hr•deg, more preferably up to 0.02 kcal/m•hr•deg, in thermal conductivity.

The units 6, blocks 7 and heat insulating material 11 provided in the space 10 are held between the cooling plate 1 and the radiating plate 8 by fasteners, such as bolts and nuts, for fastening the two plates 1, 8 at their end portions. To prevent conduction of heat through the fasteners, it is desirable to interpose heat insulating washers or the like.

Radiating fans 12 are installed on the rear side of the radiating plate 8. Although the radiating fan 12 is disposed at each of upper and lower two locations in the illustrated case, the position and number of fans to be installed are not limited specifically.

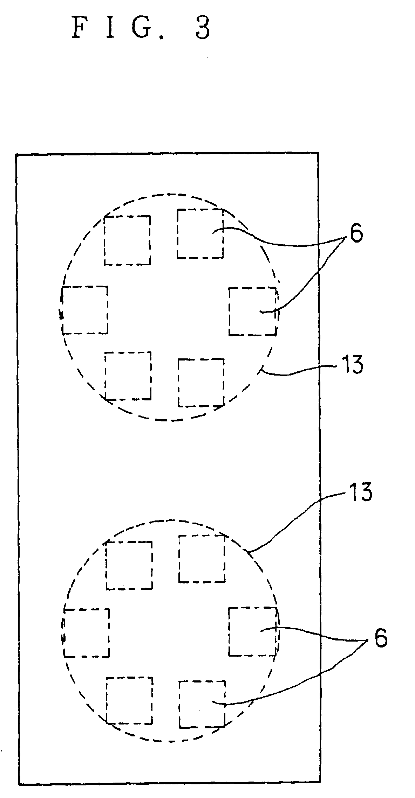

FIG. 3 shows the relationship between the regions 13, 13 to be exposed to a current of air from the radiating fans 12, 12 and the position of the electronic units 6 installed.

According to the present invention, it is desired to arrange the electronic cooling units 6 in the above regions 13, 13 to enable the radiating side of the units 6 to give off heat efficiently. FIG. 3 shows six units 6 arranged in each region 13 and spaced apart equidistantly circumferentially thereof.

Usually an axial blower of the open type is used as the radiating fan 12. The region 13 is circular in this case, whereas the region can be square depending on the type of fan insofar as electronic cooling units 6 are arranged in the region 13 to be exposed to a current of air.

The speed of rotation of the radiating fan 12 is controllable in accordance with the temperature of the cooling plate 1. For example, it is driven at a high speed when starting cooling and slowed down upon the temperature reaching a steady-state level, whereby the power consumption and noise can be diminished. A known control method is usable, for example, with use of a temperature sensor provided on the cooling plate 1.

With the cooler of the present invention, the space 10 of at least 5 mm is provided between the cooling plate 1 and the radiating plate 8, and the clearance l0a in the space 10 is packed with the heat insulating material 11. This prevents heat transfer between the two plates 1, 8 through the clearance lOa to minimize the heat flow loss. If the space 10 is less than 5 mm, the heat insulating material 11 fails to produce a sufficient heat insulating effect, whereas if the space is conversely excessive, a greatly improved heat insulating effect is not expectable, merely with an increased dimension given to the cooler in the direction of its thickness. To be satisfactory, therefore, the space 10 is about 5 to about 7 mm.

Further according to the invention, the electronic cooling units 6 are arranged in the regions 13 to be exposed to a current of air from the radiating fans 12. This assures efficient removal of heat from the radiating side of the units 6 through the radiating plate 8.

Although the electronic cooling element mounted in the unit 6 becomes less efficient with an increase in the temperature difference between the heat absorbing face and the radiating face, the present invention enables the radiating side of the unit to give off heat efficiently, consequently permitting the electronic cooling element, namely, the unit 6, to retain a high efficiency for a prolonged period of time.

The present invention minimizes the heat flow loss that would occur between the cooling plate 1 and the radiating plate 8, further permits the electronic cooling units 6 to retain a high efficiency for a prolonged period of time and therefore enables the cooler to exhibit improved performance.

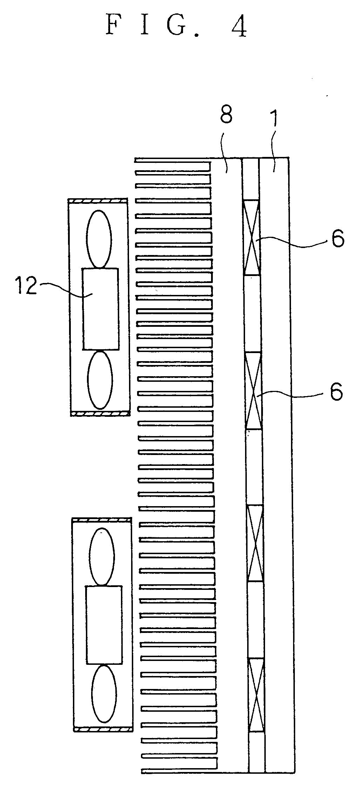

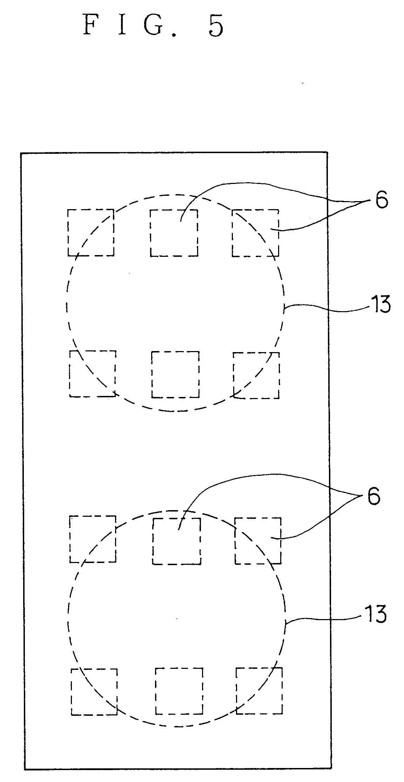

The cooler of the present invention and a comparative cooler shown in FIGS. 4 and 5 were subjected to a cooling efficiency comparative test with the following result. Incidentally, throughout FIGS. 1 to 5, like parts are designated by like reference numerals.

EP 0 653 215 A1

|  | Device of the Invention | Comparative Device |
|---|---|---|
| Bag | 18 x 29 cm, made of polyvinyl chloride, 0.38 mm in film thickness, 1.24 mm in bag thickness, about 25 mm in channel width, 36.7 ml in channel volume. | |
| Cooling plate | Made of aluminum (20 x 30 cm). | |
| Heat insulating material | Polyurethane foam, 0.017 kcal/m·hr·deg in thermal conductivity. | —— |
| Blocks | Made of copper (about 3.5 mm in thickness), 0.98 cal/cm·sec·deg in thermal conductivity. | —— |
| Radiating fans | Two units. | |
| Cooling units | Manufactured by Thermovonics Co., Ltd., 12 units (T-11D114-QDO). | |
| Arrangement of units | Six units arranged at equal spacings circumferentially of the fan (see FIG. 3). | Four rows each comprising three units arranged at a spacing of about 8 cm (see FIG. 5). |
| Power supply voltage for units | 98 V | |
| Fin | Made of aluminum (A-5052). Fins of 4 cm in height and 0.8 mm in thickness are disposed on a plate of 30 x 40 cm at a pitch of 4 mm.(total number=98) | |
| Ambient temp. | 35°C (constant-temperature air chamber) | |
| Temp. of circulating water | 37°C (constant-temperature water bath) | |
| Flow rate of circulating water | 30 ml/min | |

Cooling unit:  22.5 mm x 25 mm x 2.9 mm in size,
31.7 W in maximum heat absorption (TC=20°C).

Procedure

(1) The device of the invention and the comparative device were installed in a constant-temperature air chamber.

(2) A bag was attached to each device, and a circuit, pump, etc. were so set that water in a constanttemperature bath circulated through the bag at a constant flow rate.

(3) The temperature of the circulating water was measured at the bag outlet at an interval of 3 minutes after the start of cooling.

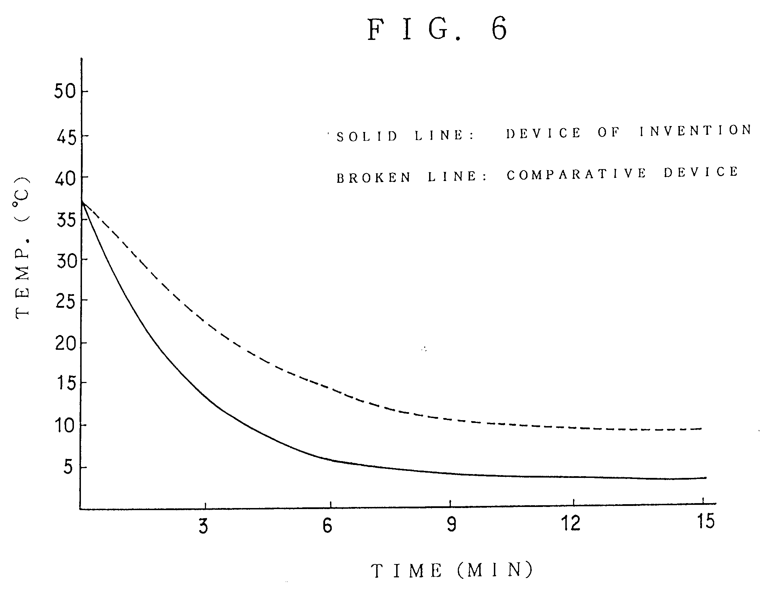

The graph of FIG. 6 reveals that about 9 minutes after the start of cooling, the device of the invention cooled the circulating water to 4 °C, but that the comparative device was unable to cool the water to not higher than 9 °C. Further although the device of the invention maintained a constant cooling temperature, the comparative device exhibited slight temperature variations.

The cooler of the present invention for plasma cooling bags comprising electronic cooling units as cooling means is so construced as to prevents heat conduction between the radiating side and the cooling side to the greatest possible extent and to fully exhibit the effect of the radiating fans. Consequently, the device can be compacted and is adapted to attain a predetermined temperature in a shortened period of time and to maintain the predetermined temperature with improved stability without entailing an increased cost or an increase in the priming volume due to an elongated plasma channel. The present invention is useful especially for cooling plasma cooling bags in practicing cryofiltration.

## Claims

1. A cooler for plasma cooling bags comprising a multiplicity of electronic cooling units dispersedly arranged between a cooling plate and a radiating plate for cooling the plasma cooling bag by the cooling plate and removing heat from the radiating plate by radiating fins provided on the radiating plate and a radiating fan for applying a current of air to the fins, the cooler being characterized in that:
   (i) a space of at least 5 mm is provided between the cooling plate and the radiating plate,
   (ii) the electronic cooling units have a heat absorbing side fastened to the cooling plate with a thermally conductive block interposed therebetween and a radiating side joined directly to the radiating plate, or the electronic cooling units have their heat absorbing side joined directly to the cooling plate and their radiating side fastened to the radiating plate with a thermally conductive block interposed therebetween, and
   (iii) a heat insulating material is filled in a clearance between the cooling plate and the radiating plate.

2. A cooler for plasma cooling bags as defined in claim 1 wherein at least some of the electronic cooling units are arranged in a region to be exposed to the current of air applied by the fan to the radiating plate.

3. A cooler for plasma cooling bags as defined in claim 2 wherein of the total area occupied by the electronic cooling units, the combined area of the units arranged in the region to be exposed to the current of air applied to the radiating plate by the fan corresponds to at least 10% of the area of the cooling plate.

4. A cooler for plasma cooling bags as defined in claim 1 wherein the thermally conductive block is at least 0.8 cal/cm•sec•deg in thermal conductivity.

5. A cooler for plasma cooling bags as defined in claim 1 wherein the heat insulating material is up to 0.03 kcal/m•hr•deg in thermal conductivity.

6. A cooler for plasma cooling bags as defined in any one of claims 1 to 5 by which the bag can be cooled to 4 °C.

F I G.  1

# F I G. 2

# F I G. 3

F I G. 4

# FIG. 5

# FIG. 6

SOLID LINE:    DEVICE OF INVENTION

BROKEN LINE:   COMPARATIVE DEVICE

TEMP. (°C)

TIME (MIN)

EP 0 653 215 A1

F I G. 7

EP 0 653 215 A1

<table>
<tr><td colspan="2"><strong>INTERNATIONAL SEARCH REPORT</strong></td><td>International application No.<br>PCT/JP94/01136</td></tr>
</table>

| | |
|---|---|
| **A. CLASSIFICATION OF SUBJECT MATTER** | |

Int. Cl$^6$  A61M1/34

According to International Patent Classification (IPC) or to both national classification and IPC

**B. FIELDS SEARCHED**

Minimum documentation searched (classification system followed by classification symbols)

Int. Cl$^5$  A61M1/00-1/38, A61J1/00-3/10

Documentation searched other than minimum documentation to the extent that such documents are included in the fields searched

| Jitsuyo Shinan Koho | 1926 – 1994 |
|---|---|
| Kokai Jitsuyo Shinan Koho | 1971 – 1994 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP, A, 57-192565 (Extracorporeal Medical<br>Specialties, Inc.),<br>November 26, 1982 (26. 11. 82)<br>& US, A, 4476685 | 1, 4-6<br>2-3 |
| A | JP, A, 59-164065 (Nikkiso Corp.),<br>September 17, 1984 (17. 09. 84), (Family: none) | 1-6 |

☐  Further documents are listed in the continuation of Box C.  ☐  See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| October 31, 1994 (31. 10. 94) | November 22, 1994 (22. 11. 94) |

| Name and mailing address of the ISA/<br>    Japanese Patent Office | Authorized officer |
|---|---|
| Facsimile No. | Telephone No. |

Form PCT/ISA/210 (second sheet) (July 1992)